# EUROPEAN PATENT APPLICATION

(11) **EP 4 537 744 A1**
(43) Date of publication of application: **16.04.2025**
(21) Application number: 23819834.5
(22) Date of filing: 06.06.2023
(51) Int. Cl.: A61B 5/00, A61B 5/0531

(54) **INDICATOR FOR MEASUREMENT DEVICE**

(30) Priority: 07.06.2022 JP 2022092504
(71) Applicant: Maruho Co., Ltd., Osaka 531-0071 (JP)
(72) Inventor: NOGUCHI, So, Osaka-shi, Osaka 540-0008 (JP)
(74) Representative: Kador & Partner Part mbB
(86) International application number: PCT/JP2023/020969
(87) International publication number: WO 2023/238854

(57) **Abstract**

[Abstract]

Provided is an indicator for a measurement device for measuring skin conditions of a human, which can display degrees of progress in at least two or more kinds of states. According to the present invention, provided is the indicator provided for the measurement device including: a case; a measurement auxiliary unit being slidably supported by the case; and a sensor part slidably supported by the measurement auxiliary unit, and the indicator has: a window part formed in a side surface portion of the case; a first display part being slidably supported inside the window part and connected to the measurement auxiliary unit; and a second display par slidably supported inside the window part and connected to the sensor part, and the indicator is configured in such a way that a slide direction and a slide amount of the first display part coincide with a slide direction and a slide amount of the measurement auxiliary unit, and a slide direction and a slide amount of the second display part coincide with a slide direction and a slide amount of the sensor part.

## Description

### TECHNICAL FIELD

The present invention relates to an indicator for a measurement device for measuring skin (derma) conditions of a human, which is used in a skin condition measurement device or the like and, in particular, relates to an indicator for a measurement device including two or more moving parts which can move separately from and independently of each other, the indicator being capable of displaying positions in states where the above-mentioned moving parts are present by a mechanism which is simple and highly reliable.

### BACKGROUND ART

As a device for measuring conditions of skin (derma) of a human such as a moisture content and resilience, there has been known a skin condition measurement device. In the device, the measurement of the moisture content of the skin is conducted generally by pressing electrodes consisted of a pair of a positive electrode and a negative electrode against the derma, flowing current and measuring electrical conductivity (conductance) of the derma between the electrodes. However, in the above-described method for measuring the moisture content of the skin, measurement sensibility varies depending on a contact angle and a pressing force between the electrodes and the derma, thereby leading to a problem in that accurate measurement of the moisture content of the skin with good reproducibility is difficult.

Therefore, in order to stabilize the pressing force between the electrodes and the derma, for example, as described in Japanese Patent Application Laid-Open Publication No. 2003-169787 (Patent Literature 1), there has been developed a skin moisture content measurement device in which a sensor including electrodes pressed against derma of a human is attached in a movable manner via a spring to a case and notification is made to a user when a predetermined pressing force is applied to the sensor, thereby prompting the user to measure a moisture content of the skin under a constant pressing force.

However, the skin moisture content measurement device described in Patent Literature 1 not only cannot stabilize a contact angle between the sensor and the derma but also a portion of the derma, which is pressed by the sensor, is deformed and a peripheral portion of the derma thereof over a wide range is also deformed. Therefore, resilience of a measured portion of the derma receives not only influence of change in resilience due to the deformation of the portion of the derma, which is pressed by the sensor, but also receives influence such as tensile stress, which is caused by the deformation of the peripheral portion of the derma, thereby leading to a problem in that the resilience becomes extremely unstable. In other words, the skin moisture content measurement device described in Patent Literature 1 has a problem in that even when the pressing force exerted by the sensor of the device is made constant and stabilized, without stabilizing the resilience of the derma which is required to measure a moisture content of the human, accurate measurement of the moisture content of the skin with good reproducibility cannot be made.

On the other hand, in order to stabilize the contact angle and the pressing force between the electrodes and the derma, as described in Japanese Patent Application Laid-Open Publication No. 2001-046344 (Patent Literature 2), there has been developed a skin condition measurement probe which measures a moisture content of the derma by locating annularly electrodes, which are pressed against the derma of a human, on a probe end surface having a certain area and pressing the probe end surface via a spring.

However, since in the skin condition measurement probe described in Patent Literature 2, the probe end surface on which the electrodes are located is formed by a plane having a wide are, for example, as to a site, which hardly has a plane portion and whose large portion is formed by a curved plane, like an arm of a human, even when the probe end surface is pressed to the skin, pressure distribution is caused on the probe end surface and in the electrodes and as a result, a pressing force exerted on the electrodes becomes uneven and unstable, thereby leading to a problem in that accurate measurement of the moisture content of the derma with good reproducibility cannot be made.

Furthermore, in the skin moisture content measurement device described in Patent Literature 1, there has been developed a display means by a means for displaying a relative position of the sensor including the electrodes and the case, which informs a user of an appropriate pressing force of the sensor.

However, in a case where the sensor including the electrodes is not pressed against the skin of the human at an appropriate pressing angle, despite the actually inappropriate pressing force, when the sensor moves to just a predetermined position with respect to the case, the display means described in Patent Literature 1 displays that the appropriate pressing force is reached, thereby leading to also a problem in that measurement failure caused by a pressing error of the sensor part cannot be prevented.

### CITATION LIST

### PATENT LITERATURE

Patent Literature 1: Japanese Patent Application Laid-Open Publication No. 2003-169787
Patent Literature 2: Japanese Patent Application Laid-Open Publication No. 2001-046344

### SUMMARY OF THE INVENTION

### TECHNICAL PROBLEM

Therefore, an object of the present invention is to provide an indicator for a measurement device, such as a skin condition measurement device for measuring skin (derma) conditions of a human, which can display degrees of progress in at least two kinds or more of states such as a pressing angle of a sensor part and a pressing force of the sensor part by a simple mechanism.

The present inventors have devoted themselves to earnest research as to a configuration of a measurement device such as a skin condition measurement device for measuring skin (derma) conditions of a human. As a result, the present inventors have found that by providing components which are movable in accordance with respective states upon detecting states such as a pressing angle of the sensor part and a pressing force of the sensor part, providing the above-mentioned moving parts with display parts mechanically connected thereto, and displaying the states in one window in an aligning manner, the above-mentioned problem can be solved, thereby reaching completion of the present invention.

In other words, according to the present invention, provided is an indicator provided for a measurement device which includes: a case; a measurement auxiliary unit slidably supported by the case; and a sensor part slidably supported by the measurement auxiliary unit is configured to have: a window part formed in a side surface portion of the case; a first display part slidably supported inside the window part and connected to the measurement auxiliary unit; and a second display part slidably supported inside the window part and connected to the sensor part, and the indicator is configured in such a way that a slide direction and a slide amount of the first display part coincide with a slide direction and a slide amount of the measurement auxiliary unit and a slide direction and a slide amount of the second display part coincide with a slide direction and a slide amount of the sensor part.

In the indicator for a measurement device according to the present invention, as with, for example, a skin condition measurement device, in a case where the measurement device has two moving parts, such as a sensor part for measuring skin conditions of a subject and a measurement auxiliary unit for guiding the sensor part, separately from and independently of each other, by providing the indicator which includes separately the first display part for displaying movement of the measurement auxiliary unit and the second display part for displaying movement of the sensor part, positions in the states in which the measurement auxiliary unit and the sensor part are present can be visually comprehended easily, thereby preventing human error due to the pressing-in failure and the like of the cover part of the measurement auxiliary unit and for example, in a case where the measurement is not started due to pressing-in insufficiency of the measurement auxiliary unit and the sensor part, a user can be easily notified of a cause of the failure in starting the measurement (that is, the pressing-in insufficiency). In addition, since the first display part and the second display part move mechanically in conjunction with the measurement auxiliary unit and the sensor part, the indicator has advantages in that a structure is simple, a power source is not needed, and reliability is extremely high.

It is preferable that in the indicator for the measurement device according to the present invention, each of the first display part and the second display part has regions, each of which is constituted of at least two or more kinds of display colors, display patterns, or combinations of the display colors and the display patterns.

In addition, it is more preferable that in the indicator for the measurement device according to the present invention, the first display part has: a first region being constituted of a first display color, a first pattern, or a combination of the first display color and the first pattern, the first display color, the first pattern, or the combination of the first display color and the first pattern indicating that the cover part is present in a first position with respect to the case; and a second region being constituted of a second display color, a second pattern, or a combination of the second display color and the second pattern, the second display color, the second pattern, or the combination of the second display color and the second pattern indicating that the cover part is present in a second position with respect to the case, and the second display part has: a third region being constituted of a third display color, a third pattern, or a combination of the third display color and the third pattern, the third display color, the third pattern, or the combination of the third display color and the third pattern indicating that the sensor part is present in a third position with respect to the case; and a fourth region being constituted of a fourth display color, a fourth pattern, or a combination of the fourth display color and the fourth pattern, the fourth display color, the fourth pattern, or the combination of the fourth display color and the fourth pattern indicating that the sensor part is present in a fourth position with respect to the case.

In addition, it is preferable that the indicator for the measurement device according to the present invention is configured to indicate that the sensor part is in a standby state when a combination of the first region and the third region is displayed in the window part, to indicate that the sensor part is in a contact state when a combination of the second region and the third region is displayed in the window part, and to indicate that the sensor part is in a measurement start state when a combination of the second region and the fourth region is displayed in the window part. In addition, in a case where the second display part in the window part displays concurrently the third coloring region and the fourth coloring region (a boundary between the third coloring region and the fourth coloring region), a user can be notified that the sensor part is not sufficiently pressed until a proper position and an appropriate pressing force are achieved.

According to the present invention, since the first display part which is directly connected to the measurement auxiliary unit and is sorted with at least two or more different colors and the second display part which is directly connected to the sensor part and is sorted with at least two or more different colors are displayed in one window part, a plurality of states of the skin condition measurement device can be easily discerned by the displays of the colors, the patterns, and the combination thereof, whereby human error due to pressing-in failure and the like of the cover part of the measurement auxiliary unit and the sensor part can be surely prevented.

In particular, in a case where in the indicator for the measurement device according to the present invention, the sensor part is pressed obliquely against the skin of the subject, the guide surface of the cover part of the measurement auxiliary unit interferes, whereby the leading end part of the sensor part cannot contact the skin of the subject or cannot move until at least the switch is turned ON. Therefore, the display in the window part does not proceed to the display of the combination of the second coloring region and the fourth coloring region, thereby allowing a user to be accurately notified that the sensor part is not appropriately pressed.

As to the indicator of the present invention, the measurement device may be configured such that the leading end part of the sensor part is provided with electrodes which are consisted of a pair of a positive electrode and a negative electrode for measuring a moisture content of the skin of the subject, the measurement auxiliary unit has: a slider part slidably supported by to the case; and a cover part having a guide surface to be pressed against the skin of the subject and an opening part for moving the electrodes of the leading end part of the sensor part in and out, the opening part formed in the guide surface, the cover part attachably and detachably connected to the slider part and includes: a first biasing means which biases the sensor part so as to separate the electrodes from the slider part; and a second biasing means which biases the slider part so as to separate the guide surface from the case. In addition, as long as a measurement target is derma, the skin condition measurement device can measure derma conditions, such as a moisture content and resilience, even of derma of an animal.

In addition, in this case, it is preferable that in order to enhance measurement accuracy of the moisture content of the skin of the subject, a plurality of the electrodes provided for the leading end part of the sensor part are arranged.

Furthermore, it is preferable that as to the indicator of the present invention, the measurement device is configured such that along with being pressed of the guide surface by the skin of the subject, initially, only the guide surface moves toward the case and subsequently, the guide surface and the leading end part of the sensor part move toward the case.

In the present invention, the measurement device has the above-described configuration, whereby along with being pressed of the guide surface of the cover part by the skin of the subject, before the leading end part of the sensor part contact the skin of the subject, the guide surface having a wider contact area than that of the leading end part contacts the skin of the subject. Therefore, in the present invention, since initially, the cover part is guided such that a pressing angle with respect to a skin surface of the subject is a right angle, and subsequently, when the leading end part of the sensor part contacts the skin of the subject, the leading end part of the sensor part is also guided by the cover part such that a pressing angle with respect to the skin surface of the subject is a right angle, it is made possible to measure the skin conditions of the subject in an appropriately contacting manner.

In the present invention, it is preferable that the measurement device is configured such that along with being pressed of the guide surface by the skin of the subject, after the guide surface and the leading end part of the sensor part have moved toward the case, further, only the leading end part of the sensor part moves toward the case.

In addition, as to the indicator of the present invention, it is preferable that the measurement device is configured such that along with being pressed of the guide surface by the skin of the subject, after movement in which the guide surface and the leading end part of the sensor part move toward the case, a pressing force per unit area which is exerted on the leading end part of the sensor part is same as or larger than a pressing force per unit area which is exerted on the guide surface.

According to the present invention, since after the movement of the guide surface and the leading end part of the sensor part toward the case, the skin of the subject, which is exposed in the opening part of the guide surface, is fixed by a marginal portion of the opening part, the skin of the subject comes to retain homogeneous and constant resilience. Therefore, after the above-mentioned movement, the pressing force per unit area exerted on the leading end part of the sensor part is configured to be same as or larger than the pressing force per unit area exerted on the guide surface, whereby independently of a pressing amount (the pressing force) of the guide surface, the leading end part of the sensor part comes to be able to contact preferentially the skin of the subject, which is retained to have homogeneous and constant resilience, thereby allowing the measurement device to accurately measure skin conditions.

Furthermore, as to the indicator of the present invention, it is preferable that the measurement device is configured such that a switch is attached to the base part of the case, and by movement of the leading end part of the sensor part up to a predetermined position toward the case, a posterior end portion of the sensor part turns the switch ON.

According to the present invention, since the leading end part of the sensor part of the measurement device is guided in such a way that a pressing angle against the skin surface of the subject is a right angle, the switch is turned ON in a state in which the skin of the subject is retained with homogeneous and constant resilience, and the measurement is started, derma conditions, such as a moisture content and resilience can be accurately and stably measured.

As to the indicator of the present invention, it is preferable that in the measurement device, in order to easily support and compactly arrange the first and second biasing means, the sensor part is provided with a first column part which slidably contacts the base part, the base part is provided with a second column part which slidably contacts the sensor part, and a central axis of the first column part and a central axis of the second column part are coaxially located.

Furthermore, in a case where the slider part of the measurement device has an opening part which slidably contacts the sensor part, it is more preferable that a central axis of the opening part and a central axis of the first column part of the sensor part are coaxially located.

As to the indicator of the present invention, as described above, in a case where the sensor part of the measurement device has the first column part and the base part thereof has the second column part, it is preferable that the first helical spring is used as the first biasing means, the second helical spring is used as the second biasing means, and a central axis of the first helical spring and a central axis of the second helical spring are coaxially located.

In addition, as to the indicator of the present invention, it is preferable that in the measurement device, the first helical spring is located between the base part and the sensor part, and the second helical spring is located between the base part and the slider part.

As to the indicator of the present invention, it is preferable that in the measurement device, for the purpose of extremely reducing the occurrence of a human error, the cover part of the measurement auxiliary unit is transparent or translucent so as to visually recognize the sensor part inside the cover part and a contact state of the sensor part with the skin of the subject.

### ADVANTAGEOUS EFFECTS OF THE INVENTION

According to the present invention, provided is an indicator for a measurement device, the measurement device including: a case; a measurement auxiliary unit being slidably supported by the case; and a sensor part slidably supported by the measurement auxiliary unit, the indicator provided for the measurement device, the indicator has: a window part formed in a side surface portion of the case; a first display part slidably supported inside the window part and connected to the measurement auxiliary unit; and a second display part slidably supported inside the window part and connected to the sensor part, and the indicator is configured in such a way that a slide direction and a slide amount of the first display part coincide with a slide direction and a slide amount of the measurement auxiliary unit, and a slide direction and a slide amount of the second display part coincide with a slide direction and a slide amount of the sensor part.

According to the present invention, the indicator which includes separately the first display part for displaying the movement of the measurement auxiliary unit (the cover part and the slider part) and the second display part for displaying the movement of the sensor part is provided, whereby it is made possible to easily comprehend where and in which states the measurement auxiliary unit and the sensor part are present in the positions and to prevent human error due to the pressing-in failure and the like of the cover part of the measurement auxiliary unit and the sensor part beforehand.

In addition, as to the indicator of the present invention, the measurement device may be configured such that the leading end part of the sensor part is provided with electrodes which are consisted of a pair of a positive electrode and a negative electrode for measuring a moisture content of the skin of the subject, the measurement auxiliary unit has: a slider part slidably supported by to the case; and a cover part having a guide surface to be pressed against the skin of the subject and an opening part for moving the electrodes of the leading end part of the sensor part in and out, the opening part formed in the guide surface, the cover part attachably and detachably connected to the slider part and includes: a first biasing means which biases the sensor part in such a way as to separate the electrodes from the slider part; and a second biasing means which biases the slider part in such a way as to separate the guide surface from the case.

As to the indicator of the present invention, since before the leading end part of the sensor part of the measurement device contacts the skin of the subject, the guide surface having a wider contact area than that of the leading end part contacts the skin of the subject, initially, the cover part is guided in such a way that a pressing angle against the skin surface of the subject is the right angle, subsequently, the leading end part of the sensor part is also guided by the cover part in such a way that the pressing angle against the skin surface of the subject is the right angle, and as a result, it is made possible to measure the skin conditions of the subject in the appropriately contacting manner.

In addition, as to the indicator of the present invention, since the skin of the subject exposed in the opening part of the guide surface of the measurement auxiliary unit of the measurement device is fixed by a marginal portion of the opening part, whereby independently of a pressing amount (the pressing force) of the guide surface, the leading end part of the sensor part comes to be able to preferentially contact the skin of the subject, which is retained to have homogeneous and constant resilience, thereby allowing the measurement accuracy of the skin condition to be enhanced.

### BRIEF DESCRIPTION OF THE DRAWINGS

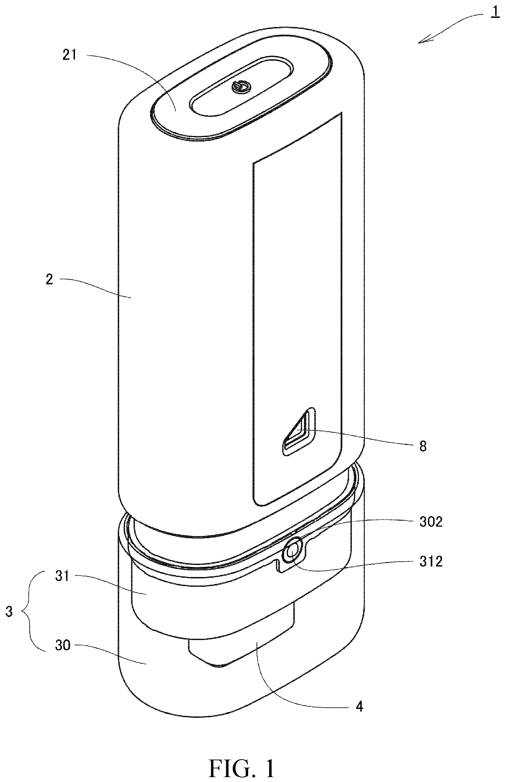

FIG. 1 is a perspective view illustrating the whole of a skin condition measurement device for which an indicator according to one embodiment of the present invention is used.

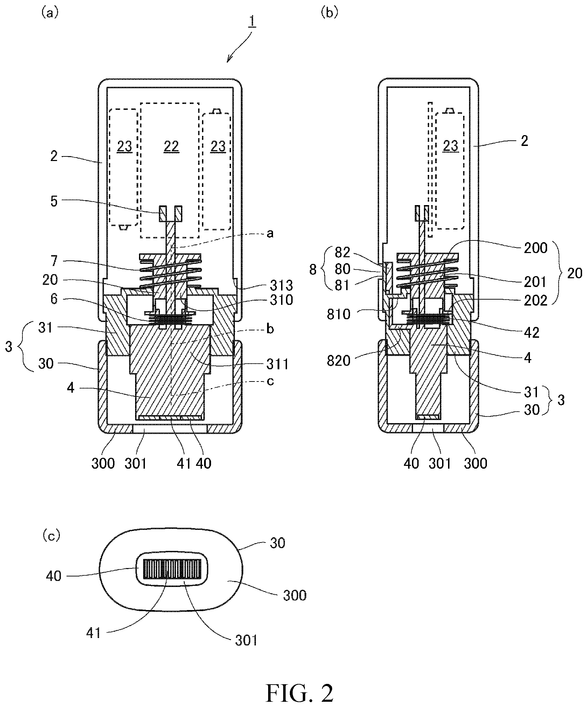

FIG. 2 shows cross-sectional views each partially illustrating the inside of the skin condition measurement device for which the indicator according to one embodiment of the present invention is used, FIG. 2 (a) is a front cross-sectional view of the skin condition measurement device, FIG. 2 (b) is a side cross-sectional view of the skin condition measurement device, and FIG. 2 (c) is a bottom cross-sectional view of the skin condition measurement device.

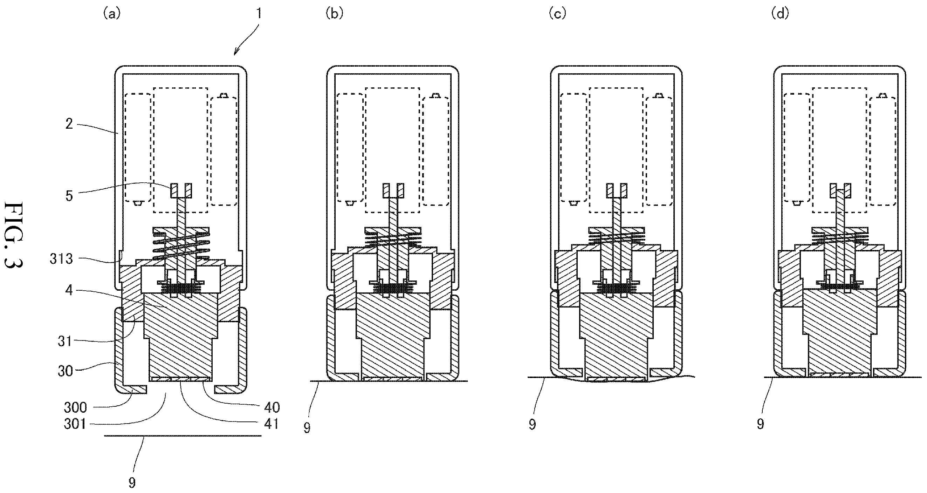

FIG. 3 shows cross-sectional views illustrating operation states of a sensor part of the skin condition measurement device for which the indicator according to one embodiment of the present invention is used, FIG. 3 (a) is a cross-sectional view in a state in which the sensor part is in a standby state, FIGS. 3 (b) and (c) are cross-sectional views in which the sensor part is in a contact state, and FIG. 3 (d) is a cross-sectional view in a state in which the sensor part is in a measurement start state.

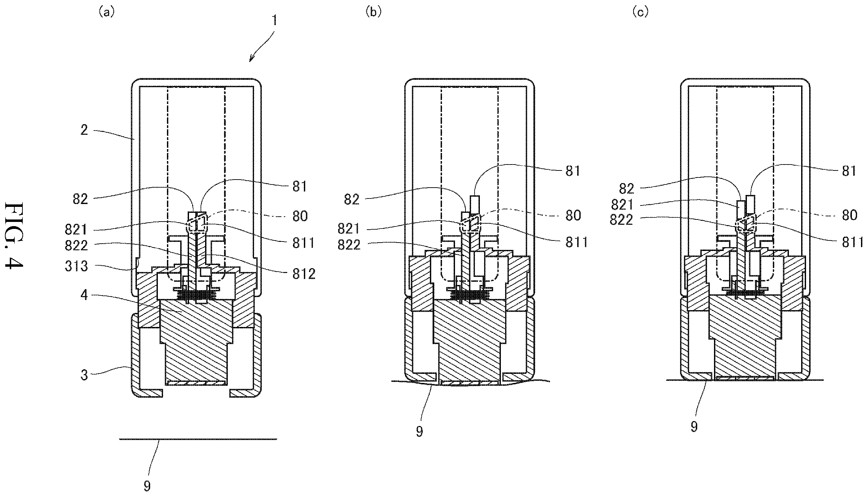

FIG. 4 shows cross-sectional views illustrating operation states of the indicator according to one embodiment of the present invention in the skin condition measurement device, FIG. 4 (a) is a cross-sectional view in a state in which the sensor part is a standby state, FIG. 4 (b) is a cross-sectional view in a state in which the sensor part is in a contact state, and FIG. 4 (c) is a cross-sectional view in a state in which the sensor part is in a measurement start state.

### DESCRIPTION OF EMBODIMENTS

Hereinafter, with reference to the accompanying drawings, an indicator for a skin condition measurement device according to one embodiment of the present invention will be described in detail. Note that the present invention is not limited to the below-described embodiment and a variety of modifications can be made without departing from the technical idea of the present invention.

### [Embodiment]

### <Skin Condition Measurement Device>

In FIG. 1, a perspective view illustrating the whole of a skin condition measurement device 1 for which an indicator 8 according to one embodiment of the present invention is used is shown. In addition, in FIG. 2, a cross-sectional view partially illustrating the inside of the skin condition measurement device 1 for which the indicator 8 according to one embodiment of the present invention is used is shown, FIG. 2 (a) is a front cross-sectional view of the skin condition measurement device 1, FIG. 2(b) is a side cross-sectional view of the skin condition measurement device 1, and FIG. 2 (c) is a bottom cross-sectional view of the skin condition measurement device 1. Hereinafter, a configuration and an operation mode of the skin condition measurement device 1 for which the indicator 8 according to one embodiment of the present invention is used will be described. Note that in the description of the present application, the "upper" as in an upper portion, an upper end, and the like means an upper side of the drawing of FIG. 2 illustrating the skin condition measurement device 1 and the "lower" as in a lower portion, a lower end, and the like means a lower side of the drawing of FIG. 2 illustrating the skin condition measurement device 1.

### [Case]

As understood with reference to FIGS. 1 and 2, the skin condition measurement device 1 of the present embodiment is a handheld measurement device having a size which allows a user to operate the device with a single hand and has a case 2 whose cross section is track-shaped, whose one end portion is open, and whose other end portion is attached with a lid part 21 which is provided with a power switch and is formed of silicon resin. Inside of the case 2, a base part 20 is fixed, and a control board 22 on which an IC chip and the like for controlling measurement of the skin condition measurement device 1 are mounted and a power source 23 such as a battery and the like for driving the control board 22 are attached. The power source 23 such as the battery inside the case 2 can be replaced by detaching a part of a side surface of the case 2. Note that a shape of the cross section of the case 2 is not necessarily to be the track-shape, and as long as the shape allows necessary components such as the control board 22 and the power source 23 to be housed and enables the single-handed operation, the shape thereof may be other general shape such as a rectangular shape, a polygonal shape, a circular shape, and an elliptical shape.

### [Base Part]

The base part 20 and the case 2 may be separate and independent components which are different from each other or the base part 20 may be formed to be integral with the case 2. The base part 20 has a first flange part 200 and a second column part 201 which extends downward from the first flange part 200 and whose whole or part is hollow or solid and is attached inside the case 2 in such a way that a central axis b of the second column part 201 coincides with a central axis of the case 2 (not shown). In addition, in a lower end portion of the second column part 201, a second flange part 202 for pressing a first biasing means 6 constituted of the later-described helical spring or the like is formed. Note that the second column part 201 is not necessarily to be of a circular column shape and as long as a shape thereof is suitable for supporting a second biasing means 7 constituted of the later-described helical spring or the like, the shape thereof may be a rectangular column shape, an elliptic column shape, or the like.

### [Slider Part]

On an inner circumferential surface of the case 2, a slider part 31 is slidably attached and a part thereof projects outward from an opening portion of the case 2. In one end portion of the slider part 31, a circular opening part 310 for slidably passing the second column part 201 of the base part 20, and in the other end portion of the slider part 34, a track-shaped opening part 311 for slidably passing the later-described sensor part 4 is formed.

### [Cover Part]

In a portion of the slider part 31, which projects outside from an opening part of the case 2, a cover part 30 is attached. In addition, the cover part 30 and the slider part 31 are attachably and detachably connected to each other by providing a small circular shaped recessed part 302 on an inner circumferential surface in the vicinity of an opening end of the cover part 30 and a small hemisphere shaped projected part 312, on a side surface of the slider part 31, which engages with the recessed part 302, thereby integrally configuring the measurement auxiliary unit 3 (see FIG. 1).

An end portion of the cover part 30 which is attached to the slider part 31 is open and in the other end portion thereof which is pressed against the skin of the subject, a flat track-shaped guide surface 300 is formed, the cover part 30 forming a cylindrical body. In addition, in the center of the guide surface 300, a small track-shaped opening part 301 for moving a leading end part 40 of the sensor part 4 in and out is formed. Note that the shape of the guide surface 300 is not necessarily to be the track-shape, and as long as an area suitable for guiding a pressing angle of the cover part 30 against the skin surface of the subject at a right angle by pressing the cover part 30 to the skin of the subject can be ensured, the shape thereof may be other general shape such as a rectangular shape, a polygonal shape, a circular shape, and an elliptical shape.

In addition, although in the present embodiment, the cover part 30 of the measurement auxiliary unit 3 is formed of a transparent or translucent material so as to be capable of visually recognizing the sensor part 4 of the cover part 30 and a contact state and the like of the sensor part 4 and the skin of the subject, the material of the cover part 30 of the measurement auxiliary unit 3 is not necessarily required to be transparent or translucent and may be opaque.

### [Sensor Part]

Inside the cover part 30 of the measurement auxiliary unit 3, the sensor part 4 is slidably supported by the opening part 311 of the slider part 31. The sensor part 4 has a first column part 42, whose whole or part is hollow or solid, in an end portion on a side on which the sensor part 4 is attached to the base part 20, has a flat track-shaped sensing surface on the leading end part 40 of the sensor part 4 which is pressed against the skin of the subject, and as a whole, a cross section thereof has a track-shaped columnar shape. Note that since the first column part 42 mainly functions to support the first biasing means 6 constituted of the later-described helical spring or the like, the first column part 42 is not necessarily to be columnar, and as long as the first column part 42 is suitable for supporting the first biasing means 6 and can slidably engage with the second column part 201 of the base part 20, a shape thereof may be a rectangular column shape, a elliptic column shape, or the like.

In addition, the skin condition measurement device 1 of the present embodiment is configured in such a way that a switch 5 is attached to the base part 20 of the case 2, the leading end part 40 of the sensor part 4 moves toward the base part 20 of the case 2 up to a predetermined position, and a posterior end portion of the sensor part 4 shields light of a photoswitch or presses the switch, thereby turning the switch 5 ON.

In the skin condition measurement device 1 of the present embodiment, in the leading end part 40 of the sensor part 4, electrodes 41 for measuring a moisture content of the skin of the subject are arranged in a comb-tooth like manner (see FIG. 2 (c)). Each of the electrodes 41 is constituted of a pair of a positive electrode and a negative electrode, and in the present embodiment, three electrodes 41 are arranged in a single horizontal row in the leading end part 40 of the sensor part 4. Thus, in the present embodiment, an area of the electrodes 41 is expanded, thereby decreasing influence of a difference in local moisture contents of skin of a subject and enhancing accuracy of measuring the moisture content. In addition, the measurement accuracy of the moisture content may be enhanced by measuring moisture contents at three points of the skin of the subject and averaging the moisture contents so as to decrease influence of variation in skin conditions due to difference in measurement sites.

The skin condition measurement device 1 of the present embodiment is suited to a measurement device of which an accurate pressing angle and pressing force are required upon measuring conditions of the skin (derma), and measurement targets thereof are not limited to a moisture content of skin. For example, although illustration is not given, in the skin condition measurement device 1 of the present embodiment, the sensor part 4 is provided with a vibration detecting element, the leading end part 40 of the sensor part 4 which is vibrated by the sensor part 4 at a predetermined frequency is brought into contact with the skin (derma) of the subject, vibration frequencies before and after contacting are detected by the vibration detecting element, and the detected frequencies are amplified by the control board, whereby the skin condition measurement device 1 can also measure resiliency of the skin (derma) based on a difference in the frequencies before and after contacting the skin (derma).

### [First Biasing Means]

**In** the skin condition measurement device 1 of the present embodiment, the first column part 42 of the sensor part 4 is slidably inserted internally inside the second column part 201 of the base part 20. **In** addition, the skin condition measurement device 1 of the present embodiment includes the first biasing means 6 which biases the sensor part 4 in such a way as to separate the leading end part 40 of the sensor part 4 from the slider part 31. In the present embodiment, as the first biasing means 6, a first helical spring 6 is used, and the first helical spring 6 is located between the second flange part 202 of the second column part 201 of the base part 20 and an upper end portion of the sensor part 4 and around the first column part 42 of the sensor part 4, thereby being supported in such a way as to be able to be pressed by the sensor part 4.

Note that although illustration is not given, in the present embodiment, by omitting the second flange part 202 of the second column part 201, the first column part 42 of the sensor part 4 can also be slidably inserted externally outside the second column part 201 of the base part 20. In this case, the first helical spring 6 is located between a lower end portion of the second column part 201 of the base part 20 and an upper end portion inside the first column part 42 of the sensor part 4 and in the first column part 42, thereby allowing the first helical spring 6 to be supported by the sensor part 4 in such a way as to be able to be pressed.

### [Second Biasing Means]

In the skin condition measurement device 1 of the present embodiment, the second column part 201 of the base part 20 slidably passes in the opening part 310 in an upper portion of the slider part 31. In addition, the skin condition measurement device 1 of the present embodiment includes the second biasing means 7 which biases the slider part 31 of the measurement auxiliary unit 3 in such a way as to separate the guide surface 300 of the cover part 30 of the measurement auxiliary unit 3 from the case 2. In the present embodiment, as the second biasing means 7, a second helical spring 7 is used, and the second helical spring 7 is located between the first flange part 200 of the second column part 201 of the base part 20 and an upper end portion of the slider part 31 of the measurement auxiliary unit 3 and around the second column part 201 of the base part 20, thereby being supported by the slider part 31 in such a way as to be able to be pressed by the slider part 31 of the measurement auxiliary unit 3.

In the skin condition measurement device 1 of the present embodiment, in order to easily support and compactly arrange the first helical spring 6 and the second helical spring 7, a central axis a of the first column part 42 of the sensor part 4 is located coaxially with a central axis b of the second column part 201 of the base part 20, and furthermore, a central axis c of the opening part 310 in an upper portion of the slider part 31 of the measurement auxiliary unit 3 is located coaxially with the central axis b of the second column part 201 of the base part 20 and the central axis a of the first column part 42 of the sensor part 4.

Thus, a central axis (not shown) of the first helical spring 6 attached around the first column part 42 is also located coaxially with a central axis (not shown) of the second helical spring 7 attached around the second column part 201, thereby allowing the skin condition measurement device 1 to have a handy and extremely compact size.

Note that in the present embodiment, a material used for the case 2, the base part 20, the cover part 30, the slider part 31, and the sensor part 4 is not particularly limited, and a resin material, a metal material, and a composite material in which the resin material and the metal material are combined can be used, and in view of light weight, excellent moldability, and also excellent insulation properties, it is preferable that a hard plastic material is used. In addition, in the present embodiment, a material used for the first biasing means 6 and the second biasing means 7 is not particularly limited, a metal material having elasticity, a resin material having elasticity, and a composite material in which the metal material and the resin material are combined can be used, and in view of excellent durability, it is preferable that the metal material having the elasticity is used.

### [Operation of Skin Condition Measurement Device]

In FIG. 3, cross-sectional views each illustrating an operation state of the sensor part 4 of the skin condition measurement device 1 for which the indicator 8 according to one embodiment of the present invention is provided are shown, FIG. 3 (a) is a cross-sectional view in which the sensor part 4 is in a standby state, each of FIG. 3(b) and FIG. 3 (c) is a cross-sectional view in which the sensor part 4 is in a contact state, and FIG. 3 (d) is a cross-sectional view in which the sensor part 4 is in a measurement start state. Hereinafter, operation of the skin condition measurement device 1 for which the indicator 8 according to one embodiment of the present invention is used will be described.

### (1) Standby State

As shown in FIG. 3 (a), when the skin condition measurement device 1 of the present embodiment is in a non-load state, that is, in a state before the guide surface 300 of the cover part 30 of the measurement auxiliary unit 3 is pressed against the skin (derma) 9 of the subject, the leading end part 40 of the sensor part 4 is biased by the first biasing means (first helical spring) 6 to a position which is most remote from the base part 20 of the case 2 and the slider part 31 of the measurement auxiliary unit 3 and the guide surface 300 of the cover part 30 is also biased by the second biasing means (second helical spring) 7 to a position which is most remote from the base part 20 of the case 2. At this time, since the leading end part 40 of the sensor part 4, which includes the electrodes 41, is held in a state in which the leading end part 40 is housed in the cover part 30, the leading end part 40 of the sensor part 4 is protected by the cover part 30 from damage and the like and malfunction due to unexpected contact of the leading end part 40 is also prevented.

### (2) Contact State-1 to Skin

As shown in FIG. 3 (b), when in the skin condition measurement device 1 of the present embodiment, the guide surface 300 of the cover part 30 of the measurement auxiliary unit 3 is pressed against skin 9 of a subject, initially, only the guide surface 300 moves toward the base part 20 of the case 2, and subsequently, the guide surface 300 and the leading end part 40 of the sensor part 4 move toward the base part 20 of the case 2.

As a result, since in the skin condition measurement device 1 of the present embodiment, before the leading end part 40 of the sensor part 4 contacts skin 9 of a subject, the guide surface 300 having a wider contact area than that of the leading end part 40 contacts skin 9 of a subject, initially, the cover part 30 of the measurement auxiliary unit 3 is guided in such a way that a pressing angle against a skin surface of a subject is a right angle, and subsequently, the leading end part 40 of the sensor part 4 also contacts the skin 9 of the subject in a state in which the leading end part 40 of the sensor part 4 is guided by the cover part 30 of the measurement auxiliary unit 3 in such a way that a pressing angle against the skin surface of the subject is a right angle. Thus, the skin condition measurement device 1 of the present embodiment can measure a skin condition (a moisture content of the skin) of the subject at an appropriate contact angle.

### (3) Contact State-2 to Skin

The skin condition measurement device 1 of the present embodiment is set such that after the leading end part 40 of the sensor part 4 has contacted the skin 9 of the subject, that is, after the guide surface 300 of the cover part 30 of the measurement auxiliary unit 3 has been pressed against the skin 9 of the subject and the guide surface 300 and the leading end part 40 of the sensor part 4 have moved toward the base part 20 of the case 2, a pressing force per unit area exerted on the leading end part 40 of the sensor part 4 is same as or larger than a pressing force per unit area exerted on the guide surface 300.

Therefore, as shown in FIG. 3 (c), when after the leading end part 40 of the sensor part 4 has contacted the skin 9 of the subject and further, the guide surface 300 of the cover part 30 of the measurement auxiliary unit 3 and the leading end part 40 of the sensor part 4 is pressed against the skin 9 of the subject, the first the slider part 31 of the measurement auxiliary unit 3 moves toward the base part 20 of the case 2 and reaches a slide limit 313, thereby causing the slider part 31 to be unable to move. As a result, after the slider part 31 has reached the slide limit 313, only the leading end part 40 of the sensor part 4 comes to move toward the base part 20 of the case 2.

As described above, since after the leading end part 40 of the sensor part 4 has contacted the skin 9 of the subject and the guide surface 300 of the cover part 30 of the measurement auxiliary unit 3 and the leading end part 40 of the sensor part 4 move toward the base part 20 of the case 2, the skin 9 of the subject exposed in the opening part 301 of the guide surface 300 is fixed by a marginal portion of the opening part 301, whereby the skin 9 of the subject comes to retain homogeneous and constant resilience. Thus, independently of a pressing amount (the pressing force) of the guide surface 300, the leading end part 40 of the sensor part 4 comes to be able to contact preferentially the skin 9 of the subject, which is retained to have homogeneous and constant resilience, thereby allowing the skin condition measurement device 1 to accurately measure a skin condition (a moisture content of the skin).

### (4) Measurement Start State

As shown in FIG. 3 (d), in the skin condition measurement device 1 of the present embodiment, when after the slider part 31 of the measurement auxiliary unit 3 has reached the slide limit 313, further, the leading end part 40 of the sensor part 4 is pressed against the skin 9 of the subject, the sensor part 4 slightly moves toward the base part 20 of the case 2 and the slider part 31 of the measurement auxiliary unit 3 and has reached the slide limit, substantially at the same time, the posterior end portion of the sensor part 4 shields light of the photoswitch attached to the base part 20 of the case 2 or presses the switch, thereby turning the switch 5 ON and causes the skin condition measurement device 1 to start measurement of a skin condition (a moisture content of the skin) of the subject.

As a result, since in the skin condition measurement device 1 of the present embodiment, the leading end part 40 of the sensor part 4 is guided in such a way that a pressing angle against the skin surface of the subject is a right angle, the switch 5 is turned ON in a state in which the skin 9 of the subject is retained with homogeneous and constant resilience, and the measurement is started, the skin condition (the moisture content of the skin) can be accurately and stably measured. Note that a measurement target of the skin condition measurement device 1 of the present embodiment is not limited to the skin (derma) of a human and the skin condition measurement device 1 can measure derma conditions such as a moisture content of derma of even an animal.

### <Indicator for Measurement Device>

In FIG. 4, cross-sectional views illustrating operation states of the indicator 8 of the skin condition measurement device 1 according to one embodiment of the present invention are shown, FIG. 4 (a) is a cross-sectional view in a state in which the sensor part 4 is in a standby state, FIG. 4 (b) is a cross-sectional view in a state in which the sensor part 4 is in a contact state, and FIG. 4 (c) is a cross-sectional view in a state in which the sensor part 4 is in a measurement start state. Hereinafter, with reference to FIGS. 1, 2, and 4, a configuration and an operation mode of the indicator 8 according to one embodiment of the present invention in the skin condition measurement device 1 will be described.

### [Configuration of Indicator]

As understood with reference to FIG. 2, the skin condition measurement device 1 of the present embodiment includes the indicator 8 for displaying a relative position of the measurement auxiliary unit 3 (the cover part 30 and the sensor part 4) with respect to the case 2 in a side surface portion of the case 2. The indicator 8 includes a window part 80 which is open in the side surface portion of the case 2, a first display part 81 which is slidably supported inside the window part 80, and a second display part 82 which is adjacent to the first display part 81 and is slidably supported in the same direction in which the first display part 81 is slidably supported.

The first display part 81 is a thin and long rod-like component and is directly and integrally connected to the slider part 31 of the measurement auxiliary unit 3 via a first connecting member 810. Therefore, in the present embodiment, the indicator is configured such that a slide direction and a slide amount of the first display part 81 coincide with a slide direction and a slide amount of each of the cover part 30 and the slider part 31 of the measurement auxiliary unit 3.

**In** addition, in the first display part 81, a surface on a side exposed from the window part 80 is color-sorted by a first coloring region 811 colored by a first display color indicating that the cover part 30 of the measurement auxiliary unit 3 is present in a position (first position) in a standby state with respect to the case 2 and a second coloring region 812 colored by a second display color indicating that the cover part 30 of the measurement auxiliary unit 3 is present in a position (second position) with respect to the case 2 in a state in which the cover part 30 has reached the slide limit 313. Note that in a case where due to character of a measurement device to which the indicator 8 is applied, three or more positions which are needed to be displayed are present, the first display part 81 may be color-sorted in accordance with a number of the positions. In addition, for sorting the first and second regions, not only the display colors but also patterns or combinations of the display colors and the patterns may be used.

In addition, the first display part 81 is not necessarily required to be directly and integrally connected to the slider part 31 and may be indirectly connected by using other connecting member (not shown), which utilizes, for example, the principle of leverage in such a way as to enlarge or shrink the slide amount of the measurement auxiliary unit 3. In addition, as long as the first display part 81 is connected to the measurement auxiliary unit 3, the first display part 81 may be a component separate from and independent of the cover part 30 and the slider part 31.

The second display part 82 is also a thin and long rod-like component and is directly and integrally connected to the sensor part 4 via a second connecting member 820. Therefore, in the present embodiment, the indicator is configured such that a slide direction and a slide amount of the second display part 82 coincide with a slide direction and a slide amount of the sensor part 4.

In addition, in the second display part 82, a surface on a side exposed from the window part 80 is color-sorted by a third coloring region 821 colored by a third display color indicating that the sensor part 4 is present in a position (third position) in a standby state with respect to the case 2 and a fourth coloring region 822 colored by a fourth display color indicating that the sensor part 4 is in a position (fourth position) in a measurement start state with respect to the case 2. Note that in a case where due to character of a measurement device to which the indicator 8 is applied, three or more positions which are needed to be displayed are present, the second display part 82 may be color-sorted in accordance with a number of the positions. In addition, for sorting the third and fourth regions, not only the display colors but also patterns or combinations of the display colors and the patterns may be used.

In addition, the second display part 82 is also not necessarily required to be directly and integrally connected to the sensor part 4 and may be indirectly connected by using other connecting member (not shown), which utilizes, for example, the principle of leverage in such a way as to enlarge or shrink the slide amount of the sensor part 4. In addition, as long as the second display part 82 is connected to the sensor part 4, the second display part 82 may be a component separate from and independent of the sensor part 4.

A target to which the indicator 8 of the present embodiment is applied is not limited to the measurement device 1 for the skin conditions such as the moisture content and the resilience, as with the measurement auxiliary unit 3 (the cover part 30 and the slider part 31) and the sensor part 4, when two or more moving parts which can separately and independently move are included, the indicator 8 can be applied to other measurement device. In addition, in a case where three or more moving parts are present, display parts may be provided in accordance with a number thereof.

As described above, as long as a measurement device includes at least the case 2, the measurement auxiliary unit 3 slidably supported by the case 2, and the sensor part 4 slidably supported by the measurement auxiliary unit 3, the indicator 8 of the present embodiment can be applied to the measurement device.

Note that in the present embodiment, a material used for the first display part 81 of the indicator 8, the first connecting member 810, the second display part 82, and the second connecting member 820 is not particularly limited, a resin material, a metal material, or a composite material in which the resin material and the metal material are combined can be used, and in view of light weight and excellent moldability, it is preferable that a hard plastic material is used.

### [Operation of Indicator]

### (1) Standby State

As shown in FIG. 4 (a), the skin condition measurement device 1 of the present embodiment is in a non-load state, that is, in a state before the cover part 30 of the measurement auxiliary unit 3 is pressed against the skin (derma) 9 of the subject, the cover part 30 of the measurement auxiliary unit 3 is in the first position which is most remote from the case 2, and the first display part 81 connected to the slider part 31 of the measurement auxiliary unit 3 displays, in the window part 80, the first coloring region 811 with the first display color indicating that the cover part 30 of the measurement auxiliary unit 3 is present in the first position. On the other hand, the sensor part 4 is also present in the third position which is most remote from the case 2 and the second display part 82 connected to the sensor part 4 displays, in the window part 80, the third coloring region 821 with the third display color indicating that the sensor part 4 is in the third position.

As a result, since in the window part 80 of the skin condition measurement device 1, a combination of the first coloring region 811 and the third coloring region 821, which indicate that the sensor part 4 is in the standby state, is displayed, a user also can visually easily comprehend that the sensor part 4 is in the standby state.

### (2) Contact State to Skin

As shown in FIG. 4 (b), in the skin condition measurement device 1 of the present embodiment, when further, the cover part 30 of the measurement auxiliary unit 3 is pressed against the skin 9 of the subject, the slider part 31 of the measurement auxiliary unit 3 moves toward the base part 20 of the case 2 and reaches the second position which is the slide limit 313. Therefore, the first display part 81 connected to the slider part 31 of the measurement auxiliary unit 3 displays, in the window part 80, the second coloring region 812 with the second display color indicating that the cover part 30 of the measurement auxiliary unit 3 has reached the second position.

On the other hand, while the sensor part 4 is retaining the third position which is most remote from the case 2, the cover part 30 of the measurement auxiliary unit 3 recedes relative to the sensor part 4, thereby coming to contact the skin 9 of the subject. Therefore, the second display part 82 connected to the sensor part 4 displays, in the window part 80, the third coloring region 821 with the third display color indicating that the sensor part 4 is retained in the third position.

As a result, since in the window part 80 of the skin condition measurement device 1, the combination of the second coloring region 812 and the third coloring region 821, which indicates that the sensor part 4 is in a contact state with the skin 9 of the subject, is displayed, a user can visually comprehend easily and accurately that although the sensor part 4 is in the contact state, the sensor part 4 has not yet started measurement.

### (3) Measurement Start State

As shown in FIG. 4 (c), in the skin condition measurement device 1 of the present embodiment, since when the cover part 30 of the measurement auxiliary unit 3 is further pressed against the skin 9 of the subject, the slider part 31 of the measurement auxiliary unit 3 is retained in the second position which is the slide limit 313, the first display part 81 connected to the slider part 31 of the measurement auxiliary unit 3 displays, in the window part 80, the second coloring region 812 with the second display color indicating that the cover part 30 of the measurement auxiliary unit 3 is retained in the second position.

On the other hand, the sensor part 4 moves toward the base part 20 of the case 2 and when the sensor part 4 has reached the fourth position which is the slide limit, substantially at the same time, the posterior end portion of the sensor part 4 shields the light of the photoswitch attached to the base part 20 of the case 2 or presses a switch, thereby turning the switch 5 ON. Therefore, the second display part 82 connected to the sensor part 4 displays, in the window part 80, the fourth coloring region 822 with the fourth display color indicating that the sensor part 4 has reached the fourth position and the switch 5 is turned ON.

As a result, since in the window part 80 of the skin condition measurement device 1, the sensor part 4 turns the switch 5 ON, whereby a combination of the second coloring region 812 and the fourth coloring region 822, which indicates that measurement of a skin condition (a moisture content of skin) of the subject is started, a user can visually comprehend easily and accurately that the sensor part 4 has contacted the skin 9 of the subject and the measurement has been started. Note that in a case where the second display part 83 in the window part 80 displays concurrently the third coloring region 821 and the fourth coloring region 822 (a boundary between the third coloring region 821 and the fourth coloring region 822), a user can easily comprehend that the sensor part 4 is not sufficiently pressed until a proper position and an appropriate pressing force are achieved.

As described above, the indicator 8 for the skin condition measurement device according to one embodiment of the present invention includes separately the first display part 81 which displays the movement of the measurement auxiliary unit 3 and the second display part 82 which displays the movement of the sensor part 4, whereby the positions in the states in which the measurement auxiliary unit 3 and the sensor part 4 are present can be visually comprehended easily and accurately, human error due to pressing-in failure and the like of the cover part 30 of the measurement auxiliary unit 3 and the sensor part 4 can be prevented beforehand, and for example, in a case where the measurement is not started due to pressing-in insufficiency of the measurement auxiliary unit 3 and the sensor part 4, a user can be notified of a cause of the failure in starting the measurement (that is, the pressing-in insufficiency). In addition, since the first display part 81 and the second display part 82 move mechanically in conjunction with the measurement auxiliary unit 3 and the sensor part 4, the indicator 8 has advantages in that a structure is simple, a power source is not needed, and reliability is extremely high.

Furthermore, since in the indicator 8 of the present embodiment for a skin condition measurement device, the first display part 81 which is directly connected to the measurement auxiliary unit 3 and is color-sorted with at least two or more different colors and the second display part 82 which is directly connected to the sensor part 4 and is color-sorted with at least two or more different colors are displayed in one window part 80, a plurality of states of the skin condition measurement device 1 can be easily discerned by the displays of the colors and the combination thereof, whereby human error due to pressing-in failure and the like of the cover part 30 of the measurement auxiliary unit 3 and the sensor part 4 can be surely prevented.

In particular, in a case where in the indicator 8 of the present embodiment, the sensor part 4 is pressed obliquely against the skin 9 of the subject, the guide surface 300 of the cover part 30 of the measurement auxiliary unit 3 interferes, whereby the leading end part 40 of the sensor part 4 cannot contact the skin 9 of the subject or cannot move until at least the switch 5 is turned ON. Therefore, the display in the window part 80 does not proceed to the display of the combination of the second coloring region 812 and the fourth coloring region 822, thereby allowing a user to be accurately notified that the sensor part 4 is not appropriately pressed.

### REFERENCE SIGNS LIST

- 1: skin condition measurement device
- 2: case
- 20: base part
- 200: first flange part
- 201: second column part
- 202: second flange part
- 21: lid part
- 22: control board
- 23: power source
- 3: measurement auxiliary unit
- 30: cover part
- 300: guide surface
- 301: opening part
- 302: recessed part
- 31: slider part
- 310, 311: opening part
- 312: projected part
- 313: slide limit
- 4: sensor part
- 40: leading end part
- 41: electrodes
- 42: first column part
- 5: switch
- 6: first biasing means (first helical spring)
- 7: second biasing means (second helical spring)
- 8: indicator
- 80: window part
- 81: first display part
- 810: first connecting member
- 811: first coloring region
- 812: second coloring region
- 82: second display part
- 820: second connecting member
- 821: third coloring region
- 822: fourth coloring region
- 9: skin (derma) of subject
- a: central axis of first column part
- b: central axis of second column part
- c: central axis of opening part

## Claims

1. An indicator for a measurement device, the measurement device including:
a case;
a measurement auxiliary unit being slidably supported by the case; and
a sensor part being slidably supported by the measurement auxiliary unit, the indicator being provided for the measurement device, wherein the indicator has:
a window part being formed in a side surface portion of the case;
a first display part being slidably supported inside the window part and being connected to the measurement auxiliary unit; and
a second display part being slidably supported inside the window part and being connected to the sensor part, and
the indicator is configured in such a way that
a slide direction and a slide amount of the first display part coincide with a slide direction and a slide amount of the measurement auxiliary unit, and
a slide direction and a slide amount of the second display part coincide with a slide direction and a slide amount of the sensor part.

2. The indicator for the measurement device according to claim 1, wherein each of the first display part and the second display part has regions, each of which is constituted of at least two or more kinds of display colors, display patterns, or combinations of the display colors and the display patterns.

3. The indicator for the measurement device according to claim 2, wherein the first display part has: a first region being constituted of a first display color, a first pattern, or a combination of the first display color and the first pattern, the first display color, the first pattern, or the combination of the first display color and the first pattern indicating that the measurement auxiliary unit is present in a first position with respect to the case; and a second region being constituted of a second display color, a second pattern, or a combination of the second display color and the second pattern, the second display color, the second pattern, or the combination of the second display color and the second pattern indicating that the measurement auxiliary unit is present in a second position with respect to the case, and
the second display part has: a third region being constituted of a third display color, a third pattern, or a combination of the third display color and the third pattern, the third display color, the third pattern, or the combination of the third display color and the third pattern indicating that the sensor part is present in a third position with respect to the case; and a fourth region being constituted of a fourth display color, a fourth pattern, or a combination of the fourth display color and the fourth pattern, the fourth display color, the fourth pattern, or the combination of the fourth display color and the fourth pattern indicating that the sensor part is present in a fourth position with respect to the case.

4. The indicator for the measurement device according to claim 3, wherein the indicator is configured
to indicate that the sensor part is in a standby state when a combination of the first region and the third region is displayed in the window part,
to indicate that the sensor part is in a contact state when a combination of the second region and the third region is displayed in the window part, and
to indicate that the sensor part is in a measurement start state when a combination of the second region and the fourth region is displayed in the window part.

5. The indicator for the measurement device according to any one of claims 1 to 4, wherein in the measurement device
a leading end part of the sensor part is provided with electrodes for measuring a moisture content of skin of a subject,
the measurement auxiliary unit is configured to include:
a slider part being slidably supported by the case; and
a cover part having a guide surface to be pressed against the skin of the subject and an opening part for moving the electrodes in and out, the opening part being located in the guide surface, the cover part being attachably and detachably connected to the slider part, the measurement device including:
a first biasing means which biases the sensor part in such a way as to separate the electrodes from the slider part; and
a second biasing means which biases the slider part in such a way as to separate the guide surface from the case.

6. The indicator for the measurement device according to claim 5, wherein in the measurement device, a plurality of the electrodes are arranged.

7. The indicator for the measurement device according to claim 5, wherein the measurement device is configured such that along with being pressed of the guide surface by the skin of the subject, initially, only the guide surface moves toward the case and subsequently, the guide surface and the electrodes move toward the case.

8. The indicator for the measurement device according to claim 7, wherein the measurement device is configured such that along with being pressed of the guide surface by the skin of the subject, after the guide surface and the electrodes have moved toward the case, further, only the electrodes move toward the case.

9. The indicator for the measurement device according to claim 8, wherein the measurement device is configured such that along with being pressed of the guide surface by the skin of the subject, after movement in which the guide surface and the electrodes move toward the case, a pressing force per unit area which is exerted on the leading end part of the sensor part is same as or larger than a pressing force per unit area which is exerted on the guide surface.

10. The indicator for the measurement device according to claim 9, wherein the measurement device is configured such that
inside the case, a base part is fixed,
a switch is attached to the base part, and
by movement of the electrodes up to a predetermined position toward the case, a posterior end portion of the sensor part turns the switch ON.

11. The indicator for the measurement device according to claim 5, wherein in the measurement device,
the sensor part has a first column part which slidably contacts the base part,
the base part has a second column part which slidably contacts the slider part, and
a central axis of the first column part and a central axis of the second column part are coaxially located.

12. The indicator for the measurement device according to claim 11, wherein in the measurement device,
the slider part has an opening part which slidably contacts the sensor part, and
a central axis of the opening part and a central axis of the first column part are coaxially located.

13. The indicator for the measurement device according to claim 5, wherein in the measurement device,
a first helical spring constitutes the first biasing means and a second helical spring constitutes the second biasing means, and
a central axis of the first helical spring and a central axis of the second helical spring are coaxially located.

14. The indicator for the measurement device according to claim 13, wherein in the measurement device,
the first helical spring is located between the base part and the sensor part, and the second helical spring is located between the base part and the slider part.

15. The indicator for the measurement device according to claim 5, wherein in the measurement device, the cover part is transparent or translucent so as to visually recognize the sensor part inside the cover part.
